(19) Europäisches Patentamt
European Patent Office
Office européen des brevets

(11) Veröffentlichungsnummer: **0 003 805**
**B1**

(12) **EUROPÄISCHE PATENTSCHRIFT**

(45) Veröffentlichungstag der Patentschrift: **30.06.82**

(21) Anmeldenummer: **79100448.4**

(22) Anmeldetag: **15.02.79**

(51) Int. Cl.³: **C 07 D 237/14,**
A 01 N 43/58

# (54) Pyridazonverbindungen und diese enthaltende herbizide Zusammensetzungen.

(30) Priorität: **25.02.78 DE 2808193**

(43) Veröffentlichungstag der Anmeldung:
**05.09.79 Patentblatt 79/18**

(45) Bekanntmachung des Hinweises auf die Patenterteilung:
**30.06.82 Patentblatt 82/26**

(84) Benannte Vertragsstaaten:
**BE CH DE FR GB IT LU NL SE**

(56) Entgegenhaltungen:
**DE - A - 1 445 533**
**DE - A - 1 542 686**
**FR - A - 2 001 700**

(73) Patentinhaber: **BASF Aktiengesellschaft**
**Carl-Bosch-Strasse 38**
**D-6700 Ludwigshafen (DE)**

(72) Erfinder: **Rohr, Wolfgang, Dr.**
**Gontardstrasse 4**
**D-6800 Mannheim 1 (DE)**
Erfinder: **Hansen, Hanspeter, Dr.**
**Thorwaldsenstrasse 5**
**D-6700 Ludwigshafen (DE)**
Erfinder: **Wuerzer, Bruno, Dr. Dipl.-Landwirt**
**Wilhelm-Busch-Strasse 55**
**D-6703 Limburgerhof (DE)**
Erfinder: **Oeser, Heinz-Guenter, Dr.**
**Friedrich-Burschell-Weg 19**
**D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 003 805 B1

# Pyridazon-Verbindungen und diese enthaltende herbizide Zusammensetzungen

Die vorliegende Erfindung betrifft neue wertvolle Pyridazone, Herbizide, welche diese Verbindungen als Wirkstoffe enthalten sowie Verfahren zur Bekämpfung unerwünschten Pflanzenwuchses mit diesen Verbindungen.

Es ist bekannt, 1-Phenyl-4,5-dimethoxy-pyridazon-(6), 1-(m-Trifluormethyl-phenyl)-4,5-dimethoxy-pyridazon-(6) oder 1-(m-Tetrafluoräthoxy-phenyl)-4-methylamino-5-chlor-pyridazon(6) als Herbizide zu verwenden (DE—OS 2 526 643, BE—PS 728 164, DE—PS 1 197 676). Ihre selektive herbizide Wirkung ist unbefriedigend. Die neuen Pyridazon-Verbindungen gemäß der Erfindung haben demgegenüber eine bessere selektive herbizide Wirkung.

Es wurde gefunden, daß substituierte Pyridazone der allgemeinen Formel

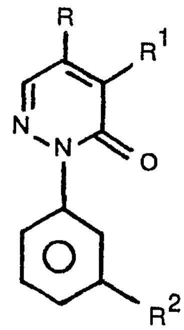

in der R und $R^1$ einen niederen Alkoxyrest, bevorzugt den Methoxyrest, $R^2$ den Tetrafluorethoxyrest oder Trifluormonobromethoxyrest bedeutet, eine gute herbizide Wirkung haben.

Die Herstellung der neuen Verbindungen kann in der Weise durchgeführt werden, daß man 4,5-Dihalogen- oder 4-Alkoxy-5-Halogenpyridazone der Formel

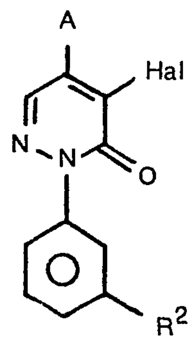

in der A einen Halogen (Cl, Br) oder Alkoxy (Methoxy) und Hal ein Halogen (Cl, Br) bedeutet und $R^2$ die oben genannten Bedeutungen hat, mit einem Alkoholat der Formel R—B oder $R^1$—B in der R und $R^1$ die oben genannten Bedeutungen haben und B Na oder K bedeutet, in Gegenwart einer organischen Flüssigkeit umsetzt.

Die als Ausgangsprodukte benötigten 4-Alkoxy-5-halogenpyridazone und 4,5-Dihalogenpyridazone sind entweder bekannt oder können in Analogie zu den in DE—PS 1 245 207, DE—PS 1 210 241 und DE—OS 2 526 643 beschriebenen Verfahren erhalten werden, beispielsweise, indem man substituierte Hydrazine wie üblich aus dem entsprechenden Diazoniumsalz durch Reduktion herstellt und ohne oder nach ihrer Isolierung als Salz mit einer 3-Formyl-2,3-dihalogen-acrylsäure in bekannter Weise zum entsprechenden Pyridazon umsetzt.

Die Isolierung der substituierten Hydrazine als Salze, z.B. als Hydrochloride vor der nachfolgenden Umsetzung zum entsprechenden Pyridazon führt jedoch zu reineren Produkten.

Die Herstellung der 4,5-Dialkoxypyridazone durch Umsetzung mit Alkoholat erfolgt bei Verwendung der entprechenden 4,5-Dihalogenpyridazone beispielsweise mit zwei Molen Alkoholat je Mol Pyridazon und bei Verwendung von 4-Alkoxy-5-halogenpyridazonen beispielsweise mit einem Mol Alkoholat je Mol Pyridazon.

Als organische Flüssigkeiten verwendet man zweckmäßig solche, die zwischen 100 und 160°C gegen Alkoholate inert sind, z.B. Toluol oder Xylol. Die Umsetzung kann im Temperaturbereich von 100 bis 160°C durchgeführt werden.

Die benötigten Vorprodukte haben folgende physikalische Daten:

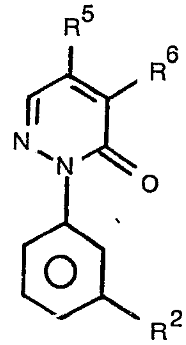

| $R^5$ | $R^6$ | $R^2$ | Schmp. (°C) |
|---|---|---|---|
| Cl | Cl | $OCF_2$—CHFBr | 67—68 |

Es werden beispielsweise folgende Endprodukte genannt.

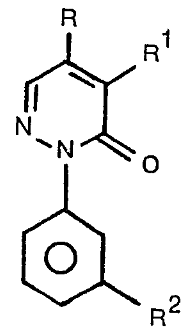

| R | $R^1$ | $R^2$ | Schmp. (°C) | $n_D^{23}$ |
|---|---|---|---|---|
| $CH_3O$ | $CH_3O$ | —$OCF_2$—$CHF_2$ | 81—83 | |
| $C_2H_5O$ | $C_2H_5O$ | —$OCF_2CHF_2$ | | 1.5177 |
| $CH_3O$ | $CH_3O$ | —$OCF_2$—CHFBr | 68—69 | |

Die erfindungsgemäßen Wirkstoffe werden beispielsweise in Form von direkt versprühbaren Lösungen, Pulvern, Suspensionen, auch hochprozentige wäßrige, ölige oder sonstige Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet. Die Anwendungsformen richten sich ganz nach den Verwendungszwecken.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate zum Beispiel Methanol. Äthanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformamid, Dimethylsulfoxid, n-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen in Betracht: Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze SULFATIERTER Hexadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylen-octylphenoläther, äthoxyliertes Isooctylphenol-, Octylphenol-, Nonylphenol, Alkylphenolpolyglykoläther, Tributylphenylpolyglykoläther, Alkylarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Superphosphat, Ammoniumsulfat, Ammoniumphosphat, Ammoniumnitrat, Harnstoffe und pflanzliche Produkte, wie Getreidemehle, Baumrinden, Holz- und Nußschalenmehl, Cellulosepulver und andere feste Trägerstoffe.

Die Formulierungen enthalten zwischen 0,1 und 95 Gewichtsprozent Wirkstoff, vorzugsweise zwischen 0,5 und 90 Gewichtsprozent.

Die neuen erfindungsgemäßen herbiziden Pyridazone können mit zahlreichen Vertretern anderer herbizider oder wachstumsregulierender Wirkstoffgruppen gemischt und gemeinsam ausgebracht werden.

Beispielsweise kommen als Mischungskomponenten Anilide, Diazine, Benzothiadiazinone, 2,6-Dinitroaniline, N-Phenyl-carbamate, Biscarbamate, Thiolcarbamate, Halogencarbonsäuren, Triazine, Amide, Harnstoffe, Diphenyläther, Triazinone, Uracile, Benzofuranderivate und andere in Betracht. Solche Kombinationen dienen zur Verbreiterung des Wirkungsspektrums und erzielen zuweilen synergistische Effekte.

## Beispiel 1

Herbizide Wirkung der neuen Verbindungen.

Der Einfluß verschiedener erfindungsgemäßer Verbindungen auf Keimung und Wachstum von unerwünschten und erwünschten Pflanzen wird in den folgenden Versuchen demonstriert. Die Versuchsserien wurden im Gewächshaus und im Freiland durchgeführt.

### I. Gewächshausversuche

Als Kulturgefäße dienten Plastikblumentöpfe mit 300 $cm^3$ Inhalt und lehmiger Sand mit etwa 1,5% Humus als Substrat. Die Samen der Testpflanzen entsprechend Tabelle 1 wurden nach Arten getrennt flach eingesät. Unmittelbar danach erfolgte bei Vorauflaufbehandlung das Aufbringen der Wirkstoffe auf die Erdoberfläche. Sie wurde hierbei in Wasser als Verteilungsmittel suspendiert oder emulgiert und mittels fein verteilender Düsen gespritzt. Nach dem Aufbringen der Wirkstoffe wurden die Gefäße leicht beregnet, um Keimung und Wachstum in Gang zu bringen und auch gleichzeitig die chemischen Verbindungen zu aktivieren. Danach deckte man die Gefäße mit durchsichtigen Plastikhauben ab, bis die Pflanzen anwuchsen. Diese Abdeckung bewirkte ein gleichmäßiges Keimen der Testpflanzen, sofern dies nicht durch die Wirkstoffe beeinträchtigt wurde.

Zum Zweck der Nachauflaufbehandlung zog man die Pflanzen je nach Wuchsform in den Versuchsgefäßen erst bis zu einer Höhe von 3 bis 10 cm an und behandelte sie danach. Eine Abdeckung unterblieb. Die Aufstellung der Versuche erfolgte im Gewächshaus, wobei für wärmeliebende Arten wärmere Bereiche (25 bis 40°C) und für solche gemäßigter Klimate 15 bis 30°C bevorzugt wurden.

Die Versuchsperiode erstreckte sich über 4 bis 6 Wochen. Während dieser Zeit wurden die Pflanzen gepflegt, und ihre Reaktion auf die einzelnen Behandlungen wurden ausgewertet. Die folgenden Tabellen enthalten die Wirkstoffe, die jeweiligen Dosierungen in kg Wirkstoff/ha und die Testpflanzenarten. Bewertet wird nach einer Skala von 0 bis 100. Dabei bedeutet 0 keine Schädigung oder normaler Aufgang und 100 kein Aufgang der Pflanzen bzw. völlige Zerstörung zumindest der oberirdischen Sproßteile.

### II. Freilandversuche

Es handelt sich um Kleinparzellenversuche auf Standorten mit lehmigem Sand vom pH 5 bis 6 und 1 bis 1,5% Humusgehalte. Es werden Vorauflaufanwendungen beschrieben, welche jeweils unmittelbar bis spätestens 3 Tage nach der Saat erfolgten. Die Kulturpflanzen wurden jeweils in Reihen gesät. Die Unkrautflora verschiedenster Artenzusammensetzung war natürlich vorkommend. Die Substanzen werden in Wasser als Träger- und Verteilermedium emulgiert oder suspendiert, mit Hilfe einer fahrbaren Parzellenspritze ausgebracht. Bei Fehlen natürlicher Niederschläge wurde künstlich beregnet, um Keimung und Wachstum von Nutzpflanzen und Unkräutern zu gewährleisten. Alle Versuche liefen über mehrere Wochen oder Monate. In diesem Zeitraum wurde in verschiedenen Zeitabständen die Bewertung nach der Skala 0 bis 100 vorgenommen.

### Ergebnis

Es zeigte sich, daß die neuen Verbindungen ein hohes Maß an herbizider Aktivität besitzen (Tab. 2, 3, 4, 5 und 6). Sie haben gegenüber bekannten Wirkstoffen entweder Vorteile hinsichtlich ihrer Wirkung gegen unerwünschte Pflanzen oder sie weisen eine deutlich bessere Verträglichkeit für Kulturpflanzen auf.

Es handelt sich um selektive herbizide Wirkstoffe zur Bekämpfung von unerwünschten Pflanzen in landwirtschaftlichen, gärtnerischen oder Forstkulturen. Ebenso können sie zur Beseitigung von Pflanzenwuchs auf Nichtkulturland z.B. auf Bahnkörpern, Parkplätzen und Industrieanlagen zur Totalunkrautbekämpfung verwendet werden. Ihre Verwendung zur Niederhaltung oder Eliminierung von krautigen unerwünschtem Pflanzenwuchs in Beerenobst-, Obst-, Nuß- und Rebanlagen ist ebenfalls möglich. Kulturpflanzen sind beispielsweise:

| Botanischer Name | Deutscher Name | Englischer Name |
|---|---|---|
| Allium cepa | Küchenzwiebel | onions |
| Ananas comosus | Ananas | pineapple |
| Arachis hypogaea | Erdnuß | peanuts (groundnuts) |
| Asparagus officinalis | Spargel | asparagus |
| Avena sativa | Hafer | oats |
| Beta vulgaris spp. altissima | Zuckerrübe | sugarbeets |
| Beta vulgaris spp. rapa | Futterrübe | fodder beets |
| Beta vulgaris spp. esculenta | Rote Rübe | table beets, red beets |
| Brassica napus var. napus | Raps | rape |
| Brassica napus var. napobrassica | Kohlrübe | |
| Brassica napus var. rapa | Weiße Rübe | turnips |
| Brassica rapa var. silvestris | Rübsen | |
| Camellia sinensis | Teestrauch | tea plants |
| Carthamus tinctorius | Saflor — Färberdistel | safflower |
| Carya illinoinensis | Pekannußbaum | pecan trees |
| Citrus limon | Zitrone | lemon |
| Citrus maxima | Pampelmuse | grapefruits |
| Citrus reticulata | Mandarine | |
| Citrus sinensis | Apfelsine, Orange | orange trees |
| Coffea arabica (Coffea canephora, Coffea liberica) | Kaffee | coffee plants |
| Cucumis melo | Melone | melons |
| Cucumis sativus | Gurke | cucumber |
| Cynodon dactylon | Bermudagras | Bermudagrass in turfs and lawns |
| Daucus carota | Möhre | carrots |
| Elaeis guineensis | Ölpalme | oil palms |
| Fragaria vesca | Erdbeere | strawberries |

| Botanischer Name | Deutscher Name | Englischer Name |
|---|---|---|
| Glycine max | Sojabohne | soybeans |
| Gossypium hirsutum (Gossypium arboreum Gossypium herbaceum Gossypium vitifolium) | Baumwolle | cotton |
| Helianthus annuus | Sonnenblúme | sunflowers |
| helianthus tuberosus | Topinambur | |
| Hevea brasiliensis | Parakautschukbaum | rubber plants |
| Hordeum vulgare | Gerste | barley |
| Humulus lupulus | Hopfen | hop |
| Ipomoea batatas | Süßkartoffeln | sweet potato |
| Juglans regia | Walnußbaum | walnut trees |
| Lactuca sativa | Kopfsalat | lettuce |
| Lens culinaris | Linse | lentils |
| Linum usitatissimum | Faserlein | flax |
| Lycopersicon lycopersicum | Tomate | tomato |
| Malus spp. | Apfel | apple trees |
| Manihot esculenta | Maniok | cassava |
| Medicago sativa | Luzerne | alfalfa (lucerne) |
| Mentha piperita | Pfefferminze | peppermint |
| Musa spp. | Obst- und Mehlbanane | banana plants |
| Nicotiana tabacum (N. rustica) | Tabak | tabacco |
| Olea europaea | Ölbaum | olive trees |
| Oryza sativa | Reis | rice |
| Panicum miliaceum | Rispenhirse | |
| Phaseolus lunatus | Mondbohne | limabeans |
| Phaseolus mungo | Urdbohne | mungbeans |
| Phaseolus vulgaris | Buschbohnen | snapbeans, green beans, dry beans |
| Pennisetum glaucum | Perl- oder Rohrkolbenhirse | |
| Petroselinum crispum spp. tuberosum | Wurzelpetersilie | parsley |
| Picea abies | Rotfichte | Norway spruce |
| Abies alba | Weißtanne | fire |

| Botanischer Name | Deutscher Name | Englischer Name |
|---|---|---|
| Pinus spp. | Kiefer | pine trees |
| Pisum sativum | Gartenerbse | English peas |
| Prunus avium | Süßkirsche | cherry trees |
| Prunus domestica | Pflaume | plum trees |
| Prunus dulcis | Mandelbaum | almond trees |
| Prunus persica | Pfirsich | peach trees |
| Pyrus communis | Birne | pear trees |
| Ribes sylvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sesamum indicum | Sesam | Sesami |
| Solanum tuberosum | Kartoffel | Irish potatoes |
| Sorghum bicolor (s. vulgare) | Mohrenhirse | sorghum |
| Sorghum dochna | Zuckerhirse | |
| Spinacia oleracea | Spinat | spinach |
| Theobroma cacao | Kakaobaum | cacao plants |
| Trifolium pratense | Rotklee | red clover |
| Triticum aestivum | Weizen | wheat |
| Vaccinium corymbosum | Kulturheidelbeere | blueberry |
| Vaccinium vitis-idaea | Preißelbeere | cranberry |
| Vicia faba | Pferdebohnen | tick beans |
| Vigna sinensis (V. unguiculata) | Kuhbohne | cow peas |
| Vitis vinifera | Weinrebe | grapes |
| Zea mays | Mais | Indian corn, sweet corn, maize |

In den Tabellen wurde die Anwendung bei Vor- und Nachauflaufbehandlung dokumentiert. Selbstverständlich können außer der Oberflächenspritzung (surface application) die Mittel auch in den Boden eingearbeitet werden. Dabei kann es sich bei Kulturpflanzenbeständen um eine Einarbeitung vor der Saat, nach der Saat oder zwischen bereits stablierten Nutzpflanzen handeln.

Auch Spezialanwendungen wie die Unterblattspritzung (post directed, lay-by) kommen in Frage, Hierbei wird der Spritzstrahl so gelenkt, daß die Blätter aufgelaufener, empfindlicher Kulturpflanzen nach Möglichkeit nicht getroffen werden, während die Mittel auf die darunterliegende Bodenfläche oder dort wachsende unerwünschten Pflanzen gelangen.

In Anbetracht der Vielseitigkeit der Applikationsmethoden können die erfindungsgemäßen Mittel oder diese enthaltende Mischungen außer bei den in den Tabellen aufgeführten Nutzpflanzen noch in einer weiteren großen Zahl von Kulturen zur Beseitigung unerwünschten Pflanzenwuchses eingesetzt werden. Die Anwendungskonzentrationen können dabei von 0,1 bis 15 kg/ha und mehr betragen je nach dem bekämpfungsobjekt.

TABELLE 1

Liste der Testpflanzen

| Botanischer Name | Abkürzung in Tabelle | Deutsche Bezeichnung | Englische Bezeichnung |
|---|---|---|---|
| Abutilon theophrasti | Abutilon theo. | Chinesischer Hanf | velvet leaf |
| Alopecurus myosuroides | Alepec. myos. | Ackerfuchsschwanz | slender foxtail |
| Amaranthus retroflexus | Amar. retr. | Zurückgekrümmter Fuchsschwanz | redroot pigweed |
| Avena fatua | Avena fat. | Flughafer | wild oats |
| Chenopodium album | Chenop. alb. | Weißer Gänsefuß | lambsquarters (goosefoot) |
| Chrysanthemum segetum | Chrys. seg. | Saatwucherblume | corn marigold |
| Ipomoea spp. | Ipom. spp. | Prunkwindearten | morningglory |
| Datura stramonium | Datura stram. | Gemeiner Stechapfel | jimsonweed |
| Echinochloa crus galli | Echin. c.g. | Hühnerhirse | barnyardgrass |
| Euphorbia geniculata | Euph. gen. | Südamerikanische Wolfsmilchart | South american member of the spurge family |
| Galium aparine | Galium apar. | Klettenlabkraut | catchweed bedstraw |
| Glycine max | glyc. max | Soja | soybeans |
| Gossypoum hirsutum | Gossyp. hirs. | Baumwolle | cotton |
| Matricaria spp. | Matric. spp. | Kamillearten | chamomile |
| Oryza sativa | Oryza sat. | Reis | rice |
| Polygonum persicaria | Polyg. pers. | Flohknöterich | ladystumb |
| Portulaca oleracea | Port. oler. | Portulak | purslane |
| Sida spinosa | Sidaspin. | | teaweed (prickly sida) |
| Sinapis alba | — | Weißer Senf | white mustard |
| Solanum nigrum | Solan. nigr. | Schwarzer Nachtschatten | black nightshade |
| Stellaria media | — | Vogelsternmiere | chickweed |
| Veronica spp. | — | Ehrenpreisarten | speedwell |

TABELLE 2

Herbizide Wirkung und Kulturpflanzenverträglichkeit der Wirkstoffe bei Vorauflaufanwendung im Gewächschaus

Grundstrukttur

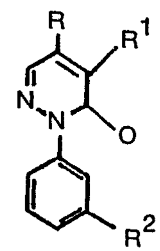

| Wirkstoff Nr. | R | $R^1$ | Substitutionen $R^2$ | kg/ha | Testpflanzen und % Schädigung Glyc. max | Gossyp. hirs. | Oryza sat. | Abutilon theo. | Alopec. myos. | Amaranth. rotr. | Chrys. seg. | Datura stram. | Ipom. spp. | Matric. spp. | Sida spin. | Echin. | Solan. nigr. | Stellaria media |
|---|---|---|---|---|---|---|---|---|---|---|---|---|---|---|---|---|---|---|
| 1 | $OCH_3$ | $OCH_3$ | $-OCF_2-CHF_2$ | 0,5 | — | 0 | 10 | 100 | 92 | 98 | 100 | 95 | 85 | 100 | 98 | 55 | 98 | 100 |
| | | | | 1,0 | — | 5 | 20 | 100 | 95 | 98 | 100 | 95 | 90 | 100 | 98 | 80 | 98 | 100 |
| | | | | 2,0 | — | 10 | 60 | — | 95 | 95 | 100 | — | 100 | 100 | 100 | 95 | 95 | 100 |
| A bekannt | $OCH_3$ | $OCH_3$ | H | 0,5 | 15 | 40 | 20 | 90 | 52 | 78 | 95 | 89 | 66 | 100 | 53 | 98 | 92 | 94 |
| | | | | 1,0 | 15 | 55 | 20 | 100 | 72 | 93 | 100 | 94 | 100 | 100 | 69 | 98 | 98 | 98 |

0 = keine Schädigung
100 = Pflanzen abgestorben

TABELLE 3

Herbizide Wirkung und Kulturpflanzenverträglichkeit neuer Wirkstoffe bei Vorauflaufanwendung im Freiland

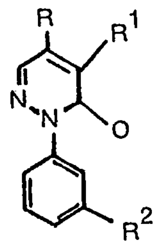

| Wirkstoff Nr. | Substitutionen R | $R^1$ | $R^2$ | kg/ha | Testpflanzen und % Schädigung Gossyp. hirs. | Amar. retr. | Chenop. alb. | Echin. c.g. | Polyg. pers. |
|---|---|---|---|---|---|---|---|---|---|
| 1 | $OCH_3$ | $OCH_3$ | $—OCF_2—CHF_2$ | 1,0 | 1 | 67 | 84 | 51 | 30 |
| | | | | 2,0 | 11 | 93 | 95 | 78 | 70 |
| A | $OCH_3$ | $OCH_3$ | H | 1,0 | 20 | 90 | 92 | 37 | 10 |
| bekannt | | | | 2,0 | 55 | 100 | 99 | 68 | 56 |
| B | 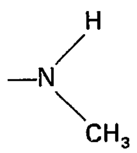 | Cl | $—OCF_2—CHF_2$ | 1,0 | 19 | 72 | 35 | 34 | 2 |
| bekannt | | | | 2,0 | 36 | 92 | 60 | 58 | 12 |

0 = keine Schädigung 100 = Pflanzen nicht aufgelaufen oder abgestorben

TABELLE 4

Herbizide Wirkung neuer Wirkstoffe bei Nachauflaufanwendung im Gewächshaus

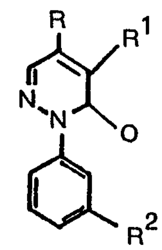

| Wirkstoff Nr. | R | $R^1$ | Substitutionen $R^2$ | kg/ha | Avena fat. | Echin. e.g. | Testpflanzen und % Schädigung Amar. retr. | Euph. gen. | Galium apar | Port. oler. | Sida spin. | Veronica spp. |
|---|---|---|---|---|---|---|---|---|---|---|---|---|
| 1 | $OCH_3$ | $OCH_3$ | $-OCF_2-CHF_2$ | 1,0 | 100 | 98 | 100 | 100 | 95 | 100 | 100 | 100 |
| A bekannt | $OCH_3$ | $OCH_3$ | H | 1,0 | 80 | 70 | 75 | 22 | 60 | 100 | 80 | 100 |

0 = keine Schädigung 100 = Pflanzen abgestorben

TABELLE 5

Selektive herbizide Wirkung der Wirkstoffe bei Vorauflaufanwendung im Gewächshaus

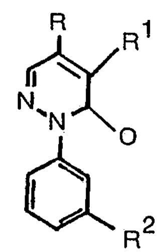

| Wirkstoff Nr. | Substitutionen R | $R_1$ | $R_2$ | kg/ha | Gossypium hirsutum | Abutilon theophrasti | Testpflanzen und % Schädigung Echinochloa crus galli | Euphorbia geniculata | Ipomoea spp. | Portulaca oleracea | Sesbania exaltata | Setaria faberii | Sida spinosa |
|---|---|---|---|---|---|---|---|---|---|---|---|---|---|
| 1 | $-OCH_3$ | $-OCH_3$ | $-OCF_2-CHF_2$ | 0,25 | 15 | 100 | 92 | 98 | 75 | 90 | 65 | 85 | 100 |
| C bekannt | $-OCH_3$ | $OCH_3$ | $CF_3$ | 0,25 | 15 | 100 | 69 | 68 | 80 | 85 | 50 | 70 | 100 |

TABELLE 6

Herbizide Wirkung der Wirkstoffe bei Nachauflaufanwendung im Gewächschaus

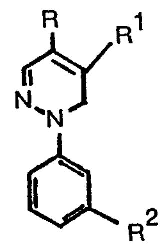

| Wirkstoff | Substitutionen R | Substitutionen $R^1$ | Substitutionen $R^2$ | kg/ha | Testpflanzen und % Schädigung: Centaurea cyanus | Echinochloa crus galli | Ipomoea spp. | Lolium multiflorum |
|---|---|---|---|---|---|---|---|---|
| 2 | $-OCH_3$ | $OCH_3$ | $-O-CF_2-CHFBr$ | 3,0 | 100 | 100 | 100 | 100 |
| 3 | $-OC_2H_5$ | $OC_2H_5$ | $-O-CF_2-CHF_2$ | 3,0 | — | 100 | 70 | 100 |

Beispiel 2

Man vermischt 90 Gewichtsteile der Verbindung 1 mit 10 Gewichtsteilen N-Methyl-α-pyrrolidon und erhält eine Lösung, die zur Anwendung in Form kleinster Tropfen geeignet ist.

Beispiel 3

20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteile des Anlagerungsproduktes von 40 Mol Äthylenoxid an 1 Mol Rizinusöl besteht. Durch Ausgießen und feines Verteilen der Lösung in 100 000 Gewichtsteilen Wasser erhält man eine waßrige Dispersion, die 0,02 Gewichtsprozent des Wirkstoffs enthält.

Beispiel 4

20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Rizinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 5

20 Gewichtsteile der Verbindung 1 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Äthylenoxid an 1 Mol Rizinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 6

20 Gewichtsteile des Wirkstoffs 2 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel 7

3 Gewichtsteile der Verbindung 3 werden mit 97 Gewichtsteilen feinteiligem Kaolin innig vermischt. Man erhält auf diese Weise ein Stäubemittel, das 3 Gewichtsprozent des Wirkstoffs enthält.

Beispiel 8

30 Gewichtsteile der Verbindung 4 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wird, innig vermischt. Man erhält auf diese Weise eine Aufarbeitung des Wirkstoffs mit guter Haftfähigkeit.

Beispiel 9

40 Gewichtsteile des Wirkstoffs 1 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

Beispiel 10

20 Teile des Wirkstoffs 2 werden mit 2 Teilen Calciumsalz der Dodecylbenzolsulfonsäure, 8 Teilen Fettalkohol-polyglykoläther, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

**Patentansprüche**

1. Substituiertes Pyridazon der allgemeinen Formel

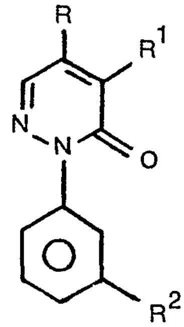

in der R und $R^1$ einen niederen Alkoxyrest, $R^2$ den Tetrafluoräthoxyrest oder Trifluormonobromäthoxyrest bedeutet.

2. 1-(3-Tetrafluoräthoxyphenyl)-4,5-dimethoxy-pyridazon-6.

3. 1-(3-Trifluormonobromäthoxyphenyl)-4,5-dimethoxy-Pyridazon-6.

4. Herbizid, enthaltend ein substituiertes Pyridazon gemäß Anspruch 1, 2 oder 3.

5. Verfahren zur Bekämpfung unerwünschten Pflanzenwuchses, dadurch gekennzeichnet, daß man den Boden oder die Pflanzen behandelt mit einem substituierten Pyridazon gemäß Anspruch 1, 2 oder 3.

**Revendications**

1. Pyridazone substituée de formule générale

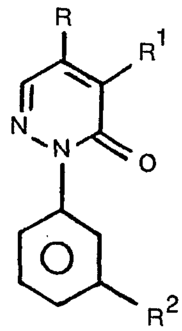

dans laquelle R et $R^1$ représentent un reste alcoxy inférieur, $R^2$ le reste tétrafluoréthoxy ou trifluoromonobrométhoxy.

2. 1-(3-tétrafluoréthoxyphényl)-4,5-diméthoxy-pyridazone-6.

3. 1-(3-trifluormonobrométhoxyphényl)-4,5-diméthoxy-pyridazone-6.

4. Herbicide contenant une pyridazone substituée selon la revendication 1, 2 ou 3.

5. Procédé de lutte contre la croissance des plantes indésirables, caractérisé par le fait que l'on traite le sol ou les plantes avec une pyridazone substituée selon la revendication 1, 2 ou 3.

**Claims**

1. A substituted pyridazone of the general formula

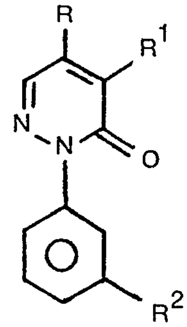

where R and $R^1$ denote lower alkoxy, and $R^2$ denotes the tetrafluoroethoxy or trifluoromonobromoethoxy radical.

2. 1-(3-tetrafluoroethoxyphenyl)-4,5-dimethoxypyridazone-6.

3. 1-(3-trifluoromonobromoethoxyphenyl)-4,5-dimethoxypyridazone-6.

4. A herbicide containing a substituted pyridazone as claimed in claim 1, 2 or 3.

5. A process for combating the growth of unwanted plants, characterized in that the soil or the plants are treated with a substituted pyridazone as claimed in claim 1, 2 or 3.